# FASCICULE DE BREVET EUROPEEN

(11) **EP 2 779 839 B1**
(45) Date de publication et mention de la délivrance du brevet: **09.01.2019**
(21) Numéro de dépôt: 12799241.0
(22) Date de dépôt: 08.11.2012
(51) Int. Cl.: A23K 20/105, A23K 20/111, A23K 20/142, A61K 36/185, A61K 31/198, A61P 9/10, A61P 9/14, A23L 33/105, A23L 33/175, A23K 20/10, A23K 50/40

(54) **PRODUITS POUR ADMINISTRATION ORALE COMPRENANT DES EXTRAITS DE GRENADE PUNICA GRANATUM, DESTINÉS À UN ANIMAL DE COMPAGNIE, ET SES APPLICATIONS**
PRODUKTE MIT GRANATAPFELEXTRAKTEN ZUR ORALEN VERABREICHUNG AN HAUSTIERE UND ANWENDUNGEN DAVON
PRODUCTS FOR ORAL ADMINISTRATION COMPRISING EXTRACTS OF PUNICA GRANATUM (POMEGRANATE), INTENDED FOR A PET, AND APPLICATIONS OF SAME

(30) Priorité: 14.11.2011 FR 1103450
(43) Date de publication de la demande: 24.09.2014
(73) Titulaire: Virbac SA, 06516 Carros (FR)
(72) Inventeur: MONGINOUX, Patricia, F-06270 Villeneuve Loubet (FR); GATTO, Hugues, F-06570 Saint Paul (FR); KARST, Christian, F-06410 Biot (FR); WALDENBERGER, Ferdinand Rudolf, 1130 Vienna (AU)
(74) Mandataire: Macquet, Christophe
(86) Numéro de dépôt international: PCT/FR2012/000447
(87) Numéro de publication internationale: WO 2013/072573

(56) Documents cités:
- EP-A1- 2 033 526
- EP-A1- 2 133 080
- WO-A1-2009/009126
- WO-A1-2010/002525
- WO-A1-2011/082080
- WO-A2-03/015695
- WO-A2-2010/012651
- KR-A- 20040 010 986
- US-A1- 2008 213 401

## Description

L'invention concerne un produit pour administration orale destiné à un animal de compagnie, comprenant des extraits de Grenade *Punica granatum*, des extraits d'Isoflavones de Soja, de la L-Carnitine et de la Taurine dans le domaine des aliments complets, des aliments complémentaires (suppléments alimentaires) ou diététiques à visée santé. Plus particulièrement, l'invention concerne un produit pour administration orale destiné, à un animal de compagnie, comprenant des extraits de Grenade *Punica granatum*, des extraits d'Isoflavones de Soja, de la L-Carnitine et de la Taurine pour la prévention, le traitement ou encore l'aide au contrôle des maladies cardiovasculaires.

L'augmentation de l'espérance de vie des animaux de compagnie tels que par exemple, les chiens et les chats, est maintenant chose commune du fait de nombreuses avancées au cours des dernières décennies, notamment dans les domaines de la nutrition animalière, des soins de santé préventive, de la médecine ou encore de la chirurgie.

Chez les chiens par exemple, les maladies cardiaques sont considérées comme la quatrième cause la plus fréquente de mortalité. Les pathologies cardiaques peuvent être classées selon qu'elles sont innées, héréditaires (maladies congénitales) ou acquises. Les maladies acquises sont principalement l'insuffisance valvulaire et la cardiomyopathie dilatée. Ces dernières peuvent toucher n'importe quelle race de chien. Néanmoins, certaines races y sont plus prédisposées que d'autres. De plus, ces maladies ont également une occurrence plus forte chez les chiens âgés.

L'insuffisance valvulaire représente jusqu'à 75% des maladies cardiaques chez le chien. Elle est ainsi la pathologie la plus fréquemment rencontrée chez cet animal.

L'insuffisance valvulaire est habituellement causée par une dégénérescence myxomateuse progressive des valves auriculo-ventriculaires qui contribue à la pathogenèse de la maladie et à une demande accrue en oxygène cellulaire. Le risque de dysfonctionnement mitral, augmente avec l'âge. Il a été démontré qu'un tiers des chiens de races prédisposées sont de petite taille et ont un murmure cardiaque caractéristique d'une insuffisance valvulaire dès l'âge de 7 ans. Les trois quarts des insuffisances cardiaques observées chez les chiens sont secondaires à cette maladie valvulaire primaire.

La cardiomyopathie dilatée (ou CMD ou DCM) est la deuxième maladie acquise la plus fréquente, après l'insuffisance valvulaire chez le chien. Elle atteint plutôt les grands chiens et peut aussi être une conséquence de l'évolution d'une insuffisance valvulaire.

Chez les chats, de telles pathologies cardiaques existent également. La pathologie cardiaque la plus courante est la cardiomyopathie hypertrophique. Cette pathologie touche majoritairement les mâles et peut apparaître à n'importe quel moment de la vie, avec une incidence majeure vers l'âge de 5-7 ans. D'autres pathologies telles que, par exemple, la cardiomyopathie dilatée et l'insuffisance cardiaque par hyperthyroïdie touchent également les chats.

Des études expérimentales (Cesselli, D. et al. Oxidative stress-mediated cardiac cell death is a major determinant of ventricular dysfunction and failure in dog dilated cardiomyopathy. Cire Res 89, 279-86 (2001*))* ont mis en évidence un rôle important du stress oxydatif dans le développement de maladies cardiovasculaires canines. Plus précisément, il a été démontré que le stress oxydatif induisait des dommages dans plusieurs types de cellules cardiovasculaires, pouvant engendrer l'initiation et la progression de maladies cardiaques.

Des données récentes suggèrent ainsi que le stress oxydatif intervient dans la régulation de l'apoptose (mort cellulaire programmée) des cardiomyocytes. Ces dérégulations sont à même de modifier la réactivité antioxydante des cellules et par conséquent, d'alourdir la sévérité de l'insuffisance cardiaque.

En effet, l'apoptose accélérée des cellules endothéliales vasculaires nuit à la vascularisation et s'additionne au développement et à la progression des maladies vasculaires. Des données récentes suggèrent que l'augmentation du taux de renouvellement des cellules endothéliales se produit en réponse à des taux augmentés de l'apoptose. Ces deux processus semblent nuire à l'intégrité de la monocouche de cellules endothéliales et causer des altérations fonctionnelles incluant une augmentation de la perméabilité vasculaire et un changement du profil de sécrétion favorisant des activités vasoconstrictrice, pro-adhésive, pro-thrombotique et pro-inflammatoire conduisant à une fibrose et à une perte progressive de l'élasticité et de l'étanchéité valvulaire.

Tenant compte de la prévalence des pathologies cardiaques chez l'animal (insuffisances valvulaires en particulier), et considérant que le stress oxydatif joue un rôle majeur dans le développement et la progression des maladies cardiaques, la possibilité de réguler ce stress oxydatif avec des composés exogènes a donc été approfondie. Les avantages de la réduction du stress oxydatif en liaison avec des anomalies physiopathologiques ont été démontrées chez l'Homme, et des efforts considérables ont été faits afin d'identifier et de caractériser des substances qui pourraient protéger les cellules endothéliales du stress oxydatif.

Toutefois, les effets des antioxydants diffèrent selon les espèces. En effet, comme le rapporte Ram, J.I. & Hiebert, L.M. Vitamin E protects porcine but not bovine cultured aortic endothelial cells from oxygen-derived free radical injury due to hydrogen peroxide. Cell Biol Toxicol 20, 55-67 (2004*)*, il apparait que les effets protecteurs conférés par des substances antioxydantes sont dépendants des espèces. Il n'est donc pas possible d'extrapoler les effets protecteurs des substances antioxydantes d'une espèce à une autre.

Il existe donc un besoin de développer un produit pour administration orale destiné à un animal de compagnie, qui repose sur la présence d'antioxydants ayant des effets protecteurs avérés sur les animaux de compagnie, de préférence sur les canidés ou sur les félidés. En particulier, il existe un besoin pour un produit permettant de protéger ou de soigner les chiens prédisposés aux maladies cardiaques, en particulier l'insuffisance valvulaire, tels les petits chiens. Il existe également un besoin pour protéger ou soigner les chiens âgés, ainsi que les grands chiens souffrant d'autres pathologies cardiaques.

En outre, il a également été montré qu'une stratégie monodimensionnelle d'utilisation d'antioxydants était insuffisante pour avoir des cardioprotecteurs efficaces. Il existe donc un besoin de développer un produit pour administration orale destiné à un animal de compagnie, comprenant une combinaison de substances dont des antioxydants et ayant des effets complémentaires ou interdépendants.

Il a été déjà montré que la L-Carnitine et la Taurine, bien connues pour leur activité antioxydante, peuvent protéger les cellules endothéliales humaines du stress oxydatif et peuvent également avoir des effets bénéfiques sur certaines cardiomyopathies du chien *(*Sanderson, S.L. Taurine and carnitine in canine cardiomyopathy. Vet Clin North Am Small Anim Pract 36, 1325-43, vii-viii (2006*)).* Ces substances sont devenues des molécules de choix à formuler dans des compléments alimentaires destinés aux chiens souffrant de problèmes cardiovasculaires.

Les composés polyphénoliques, comme les Isoflavones de Soja provenant d'extraits de soja, et les tanins extraits de Grenade, ont également été étudiés pour leur activité antioxydante et leurs effets bénéfiques potentiels sur les maladies cardiovasculaires chez l'Homme *(*Nicholson, S.K., Tucker, G.A. & Brameld, J.M. Effects of dietary polyphenols on gene expression in human vascular endothelial cells. Proc Nutr Soc 67, 42-7 (2008*)*.

Le document WO2010/002525 divulgue un produit pour administration orale comprenant des punicalagines qui sont des ellagitanins. On les trouvent notamment dans la peau de grenade et les jus de grenade. Le produit divulgué dans le document WO2010/002525 comprend en outre de L-Carnitine et de Taurine, et peut notamment être utilisé pour améliorer la santé cardiovasculaire de sujets pédiatriques.

Les documents EP2133080 et WO2010/012651 divulguent des produits pour administration orale comprenant des isoflavones de soja, des extraits de extraits de Grenade *Punica granatum,* et de la L-Carnitine.

Le document KR20040010986 décrit quant à lui un produit comprenant des extraits de extraits de Grenade *Punica granatum,* de la Taurine et de la poudre de protéines de soja.

En outre, même si la capacité antioxydante de ces quatre substances a été évaluée individuellement dans
plusieurs essais *in vitro*, leur capacité à protéger les cellules endothéliales des animaux, en particulier les cellules endothéliales canines restait jusqu'à présent inconnue et incertaine.

La solution au problème posé a pour objet un produit pour administration orale destiné à un animal de compagnie, comprenant des extraits de Grenade *Punica granatum*, caractérisé en ce qu'il comprend en outre des extraits d'Isoflavones de Soja, de la L-Carnitine et de la Taurine. Les composés antioxydants additionnels sont choisis parmi les biflavonoïdes de citron, la vitamine C (acide ascorbique) ou un de ses dérivés/sels potentiels comme l'ascorbate de calcium, la vitamine E, le coenzyme Q10 (aussi connu sous le nom de ubiquinone), les polyphénols, en particulier le resvératrol, l'extrait d'écorce du pin maritime, les extraits de thé vert, le Ginkgo Biloba, les lycopènes (par exemple le lycopène de tomate).

Les informations permettant d'évaluer les effets cytoprotecteurs des substances ont été obtenues par la mise en oeuvre d'un test sur des cellules endothéliales aortiques canines, comme cela est décrit à l'exemple 1. Les effets de la L-Carnitine, la Taurine, les extraits de Grenade et d'Isoflavones de Soja, utilisées seules ou en combinaison, ont été étudiés afin de déterminer leur potentiel dans le développement d'une stratégie de régime multidimensionnel visant à réduire l'apparition et la progression des maladies canines liées au stress oxydatif.

Les données découlant de cette étude suggèrent en particulier que les quatre substances antioxydantes que sont la L-Carnitine, la Taurine et les extraits de Grenade et d'Isoflavones de Soja sont extrêmement intéressantes dans le développement d'une stratégie de régime multidimensionnelle visant à réduire l'apparition et la progression des maladies cardiaques induites par le stress oxydatif chez les animaux de compagnie, plus précisément pour réduire la dégénérescence des cellules endothéliales impliquées dans les insuffisances valvulaires progressives.

L'invention a également pour objet un produit selon l'invention à titre d'aliment complet. Elle a aussi pour objet un produit selon l'invention à titre d'aliment complémentaire ou diététique à visée santé (suppléments nutritionnels).

Elle a également pour objet un produit selon l'invention pour le traitement ou la prévention des maladies cardiovasculaires. Elle a aussi pour objet un produit selon l'invention pour l'aide au contrôle des maladies cardiovasculaires.

L'invention a également pour objet de fournir un produit permettant d'améliorer la santé ou les conditions de vie d'un animal de compagnie, en administrant une quantité effective du produit selon l'invention.

La présente invention procure en outre une méthode pour réduire la prolifération et/ou l'apoptose des cellules endothéliales. La présente invention procure une nouvelle méthode pour protéger les cellules endothéliales du stress oxydatif. En particulier, pour protéger les cellules endothéliales canines. Ainsi, la présente invention fournit un produit pour le support de la fonction cardiaque dans le cas d'une insuffisance cardiaque chronique, en particulier chez le chien.

L'invention sera mieux comprise à la lecture de la description non limitative qui suit, rédigée au regard des figures annexées, dans lesquels :

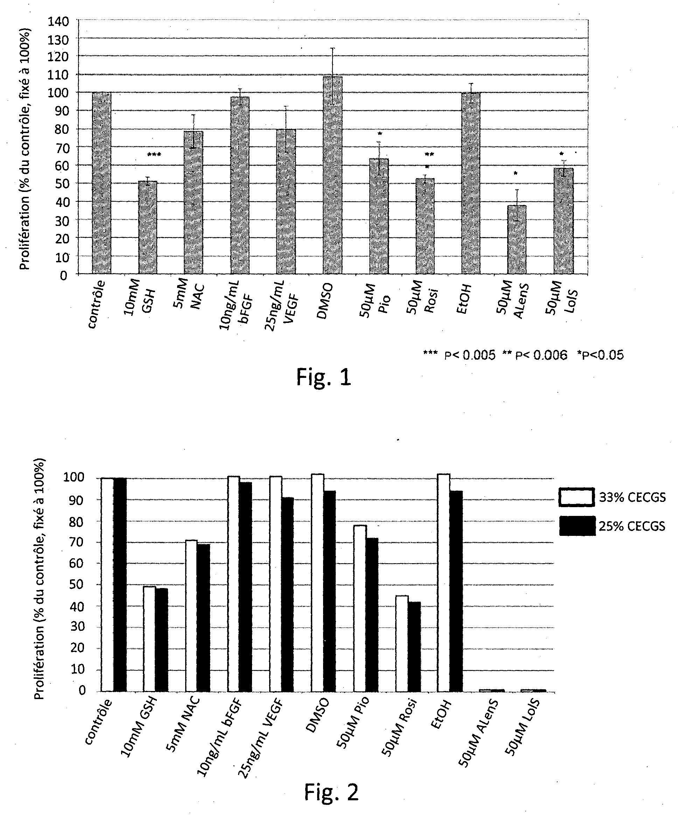

- les figures 1 à 4 représentent les résultats d'études ayant pour but d'évaluer les effets de substances de référence incluant des antioxydants (Gluthatione, GSH et N-Acétylcystéine, NAC), des facteurs de croissance vasculaire (Vascular Endothelial Growth Factor, VEGF ; basic Fibroblast Growth Factor, bFGF), d'agents sensibilisateurs des récepteurs à l'Insuline (Pioglitazone et Rosiglitazone sur la prolifération et l'apoptose de cellules endothéliales aortiques canines ;

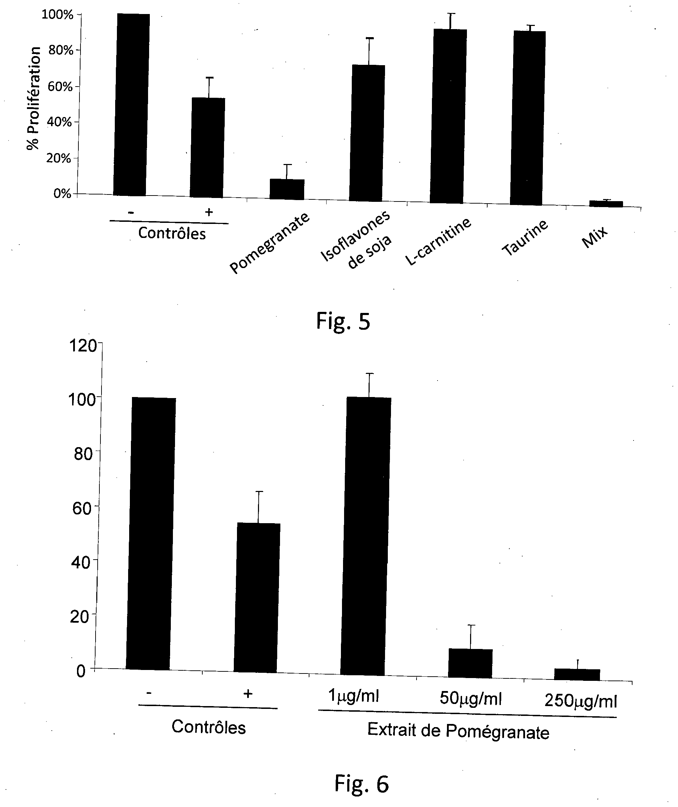

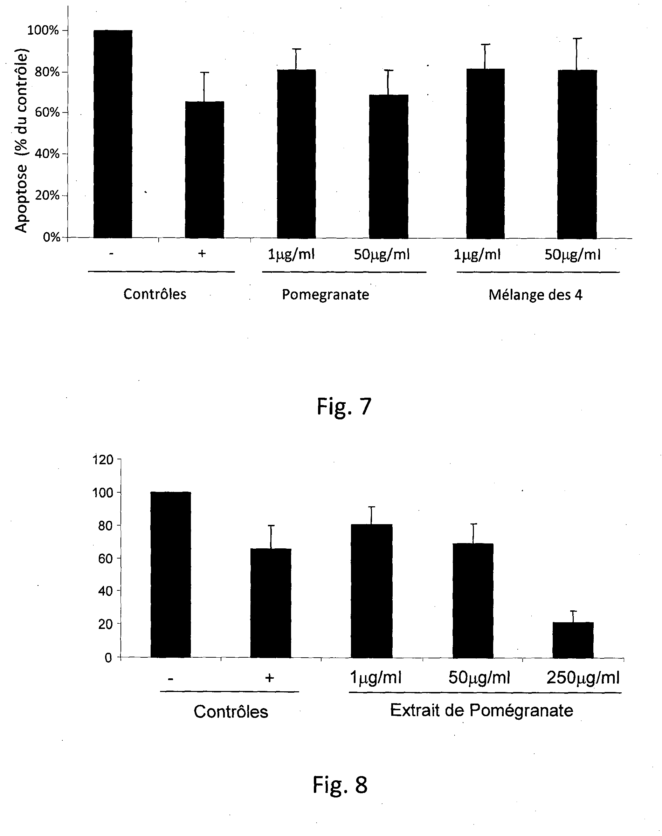

- Les figures 5 à 8 présentent les résultats d'études d'évaluation de l'effet protecteur de substances naturelles contre le stress oxydatif à l'aide d'un nouveau test développé sur des cellules endothéliales canines. Dans le cas de la figure 5, c'est l'effet sur la prolifération cellulaire qui est évalué. La figure 6 présente l'effet dose de l'extrait de pomégranate (Grenade) sur la prolifération cellulaire. Dans la figure 7, c'est l'effet sur l'apoptose des cellules endothéliales canines qui est étudié. La figure 8 présente l'effet dose de l'extrait de pomégranate (Grenade) sur l'apoptose.

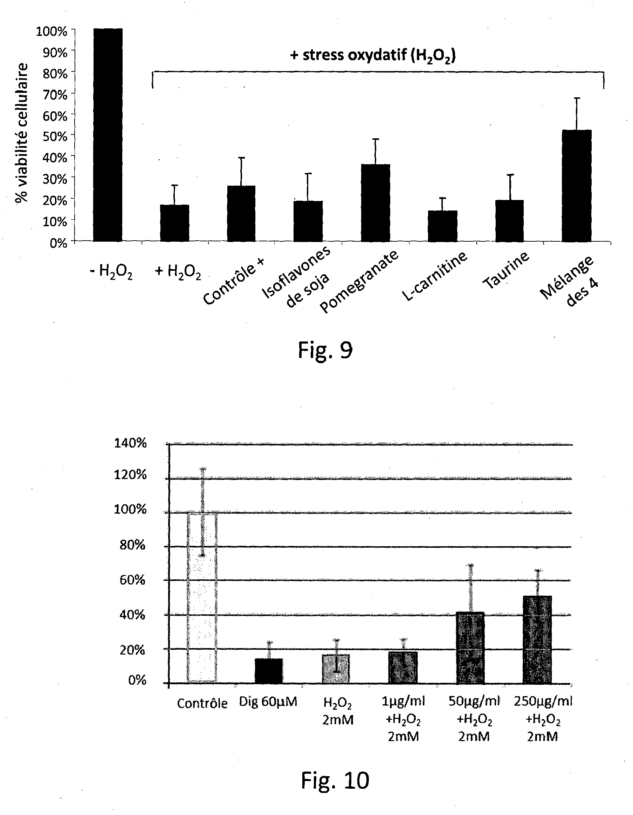

- Les figures 9 et 10 présentent les résultats d'études ayant pour but d'évaluer l'effet protecteur de substances naturelles contre la mortalité cellulaire induite par un stress oxydatif. Cette étude a été réalisée à l'aide d'un nouveau test développé sur des cellules endothéliales canines et de tests de réduction de radicaux libres; et

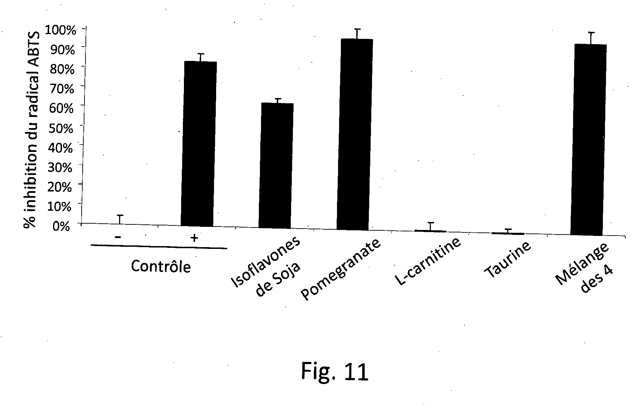

- la figure 11 présente les résultats d'une étude visant à évaluer le pouvoir antioxydant de substances naturelles par inhibition *in vitro* du radical ABTS.

Le produit pour administration orale destiné à un animal de compagnie comprend :
- des extraits de Grenade *Punica granatum*;
- des extraits d'Isoflavones de Soja;
- de la L-Carnitine; et
- de la Taurine.

Selon un mode de réalisation de l'invention, le produit comprend :
- entre 1 et 90%, entre 1 et 80%, entre 1 et 50%, entre 5 et 20% en poids du poids total du produit de L-Carnitine;
- entre 1 et 90 %, entre 1 et 80%, entre 1 et 50%, entre 5 et 30% en poids du poids total du produit de Taurine;
- entre 0,1 et 90%, entre 0,1 et 50%, entre 0,1 et 10%, entre 0,1 et 5% en poids du poids total du produit d'extraits de Grenade, *Punica granatum*; et
- entre 0,1 et 90%, entre 0,1 et 50%, entre 0,1 et 10%, entre 0,1 et 5% en poids du poids total du produit d'extraits de Soja concentrés en Isoflavones.

Selon un mode préféré de réalisation de l'invention, le produit comprend :
- entre 5 et 15 % en poids du poids total du produit de L-Carnitine;
- entre 10 et 20 % en poids du poids total du produit de Taurine;
- entre 0,1 et 5% en poids du poids total du produit d'extraits de Grenade, *Punica granatum*; et
- entre 0,5 et 5% en poids du poids total du produit d'extraits de Soja concentrés en Isoflavones.

Cependant, le choix et la quantité précise de chaque ingrédient additionnel est déterminé par l'utilisation finale du produit selon l'invention. Ce choix dépend notamment d'un ou plusieurs paramètres tels que le type d'aliment dans lequel ces ingrédients vont être ajoutés (aliment complet, aliment complémentaire, aliment diététique), la forme galénique du produit fini (gélule, comprimé, poudre, solution, etc.), l'espèce, l'âge, le poids corporel, la santé générale, le sexe, le taux de consommation, le type de maladie ou d'affection traitée, de l'animal à qui s'adresse le produit selon l'invention. Par conséquent, les quantités des ingrédients additionnels peuvent varier considérablement.

Le produit selon l'invention peut se trouver sous la forme d'un aliment complet ou d'un aliment complémentaire ou diététique à visée santé.

Le produit selon l'invention est utile pour le traitement ou la prévention des maladies cardiovasculaires, pour l'aide au contrôle des maladies cardiovasculaires, ou encore pour le support de la fonction cardiaque dans le cas d'une insuffisance cardiaque chronique.

Le produit selon l'invention est particulièrement adapté pour l'administration à un canidé ou à un félidé, en particulier un canidé sélectionné parmi les chiens de race: Caniche, Chihuahua, Bichon, Yorkshire, Cavalier King Charles, Pekingese, Pinscher, Spitz Loup, Spaniels, English Springer Spaniel, Pomeranian, Basset, Beagle, Westie, Whippet, Terriers, Fox terrier, Yorkshire Terrier.

Dans un autre aspect, le produit selon l'invention est administré quotidiennement pour une période d'au moins 1, 2, 3, 4, 5 ou 6 mois. Dans un autre aspect de l'invention, le produit est administré à une dose comprise entre 0,0001 mg/kg et 350 mg/kg. Dans un autre aspect de l'invention, le produit est sous la forme d'un comprimé, d'une gélule, d'une dragée, d'une poudre, d'un granulé, d'un sirop, d'une suspension, d'une solution, d'une émulsion.

La Grenade est le fruit du grenadier (*Punica granatum*) de la famille des Lythracées. L'extrait de Grenade possède de fortes capacités antioxydantes et anti-inflammatoires liées à la présence d'anthocyanes, de tanins ellagiques et de tannins hydrolysables. La Grenade présente également un effet protecteur de l'endothélium ainsi que vaso-relaxant. La Grenade *Punica granatum* peut être plus ou moins concentrée en punicosides et en antioxydants selon la source de grenade utilisée, ainsi que discuté à l'exemple 2, on préférera les sources de Grenade comprenant une proportion importante de punicosides, en particulier, les extraits de Grenade comprenant plus de 10, 20, 30 ou 40% de punicosides.

Les Isoflavones de Soja sont des molécules de la famille des Flavonoïdes ayant des propriétés pseudo-oestrogéniques. La Génistéine et Daidzéine sont les principaux Flavonoïdes du Soja. Les Isoflavones de Soja sont des antioxydants présentant un effet protecteur et vaso-relaxant pour l'endothélium. La Génistéine est un antioxydant capable de neutraliser les effets négatifs des radicaux libres sur les cellules endothéliales formant les vaisseaux sanguins.

La Carnitine est un composé comprenant une fonction ammonium quaternaire, elle est biosynthétisée à partir de lysine et de méthionine. Cette molécule intervient au sein de la cellule dans le transport des acides gras du cytosol vers les mitochondries lors du catabolisme des lipides dans le métabolisme énergétique. La Carnitine possède deux stéréo-isomères, sa forme biologique active est la L-Carnitine alors que la forme D est biologiquement inactive. La L-Carnitine est un antioxydant ayant également des propriétés anti-arythmiques.

La Taurine est dérivée d'un acide aminé soufré : la Cystéine. La Taurine est naturellement présente et synthétisée dans le corps. C'est un antioxydant qui intervient dans le métabolisme énergétique, dans les fonctions cardiaques et musculaires, notamment en renforçant la contractilité cardiaque, c'est-à-dire l'inotropisme.

Le produit selon l'invention se présente sous une forme galénique quelconque normalement utilisée pour une administration par voie orale, c'est-à-dire *per os*. Le produit selon l'invention se présente avantageusement sous forme solide ou sous formé liquide.

Le produit selon l'invention peut se présenter sous la forme d'un comprimé, d'une gélule, d'une dragée, d'une poudre, d'un granulé, ou encore sous forme de sirop, de suspension, de solution, d'émulsion, de pré-concentré ou de suspension de microsphères ou nanosphères ou de vésicules lipidiques ou polymériques permettant une libération contrôlée. Il peut également se présenter sous une forme incorporée dans un produit fini sous la forme d'un gel, d'une pâte, d'une croquette, d'une lamelle ou d'un jouet à mâcher tel que par exemple un os artificiel. Le produit selon l'invention est incorporé à une teneur comprise entre 0,001% et 90%, entre 0,001% et 50%, entre 0,001% et 20%, entre 0,01% et 20%, ou entre 0,01% et 10% en poids total du produit fini. La quantité de produit de l'invention dans de tels produits finis peut être déterminée par l'Homme du Métier en fonction de la destination du produit, de l'animal à traiter, de la forme du produit.

Il peut encore se présenter sous la forme d'un mélange fluide que l'on ajoutera sur ou dans une composition alimentaire. Selon le mode de réalisation de l'invention, le produit selon l'invention peut avoir une valeur nutritionnelle ou non. Ainsi, l'invention concerne aussi un aliment, qui est par exemple un aliment complet ou un aliment complémentaire (aliment complémentaire ou diététique à visée santé), pour animaux de compagnie, par exemple pour les canidés, comprenant les constituants du produit de l'invention.

De façon avantageuse, le produit selon l'invention comprend également un ou plusieurs ingrédients additionnels bien connus de l'homme du métier tels que notamment les agents appétents, les agents liants, les agents de granulation, les lubrifiants, les arômes, les colorants, les charges, les édulcorants, les émulsifiants, les exhausteurs de goût, les minéraux, les agents de pelliculage, les sels, les stabilisants, les tampons ou les vitamines. Les stabilisants comprennent les substances qui ont tendance à augmenter la durée de conservation de la composition tels que les conservateurs, les émulsifiants, les épaississants, les gaz d'emballage, les gélifiants, les humectants, les séquestrants, les synergistes ou les stabilisants. Comme agent liant, on citera par exemple les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les copolymères blocs non ioniques de polyoxyéthylène et polyoxypropylène, les monosaccharides, disaccharides et polysaccharides, les polyols ou leur mélanges. Par celluloses et leurs dérivés, on entend notamment la cellulose microcristalline, les éthers ou esters des alkyl cellulose comme la méthyl cellulose, l'éthylcellulose, l'hydroxypropylcellulose, l'hydroxypropylméthylcellulose, l'acétophthalate de cellulose, l'acétate de cellulose Comme agent diluant, on citera par exemple on citera par exemple les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les copolymères blocs non ioniques de polyoxyéthylène et polyoxypropylène, les monosaccharides, disaccharides -et polysaccharides, les polyols ou leur mélanges, les sucres comme le glucose, les maltodextrines, le sorbitol, le saccharose.

On choisira un agent appétent adapté à l'espèce cible, en particulier les carnivores domestiques tels que les chiens et les chats, on choisira, par exemple la levure de bière, les farines de viandes, les farines de poissons, les poudres de fromages ou dérivés du lait, la poudre de foie et leur mélanges, les acides aminés et leur mélanges, les arômes naturels ou artificiels.

Comme agent de granulation, on citera par exemple les copolymères de méthacrylate, l'éthylcellulose et ses dérivés. Dans un mode particulier de l'invention, on choisira le copolymère cationique de dimethylaminoethyl méthacrylate, butyl méthacrylate, et méthyl méthacrylate (connu sous le nom commercial Eudragit E100®) ou un de ses dérivés, qui pourra conférer à la composition des propriétés améliorées de résistance à l'humidité.

Comme lubrifiant, on citera par exemple l'acide stéarique et ses dérivés, l'acide citrique et ses dérivés, l'acide lactique et ses dérivés, le propylène glycols, la glycérine, les phthalates et leurs dérivés, les adipates et leurs dérivés, les sébaçates et leurs dérivés, les polyéthylène glycols et leurs dérivés, le glyceryl behenate.

Comme exhausteur de goût, on citera par exemple le sucrose et les dérivés du sucre tels que le saccharose, le sucralose.

Dans un mode alternatif de réalisation de l'invention, d'autres agents peuvent également être ajoutés pour leur propriétés bénéfiques sur le système cardiaque et vasculaire (effets inotrope, anti-arythmique, hypotenseur, vasodilatateur...), tels que: l'acide folique ou vitamine B9, les acides gras oméga-3, des métaux comme le sélénium ou le magnésium, l'extrait de crataegus Oxycantha, la L-Arginine.

Le produit selon l'invention est destiné à un animal de compagnie, c'est-à-dire un animal ayant fait l'objet d'une domestication. De préférence, l'animal de compagnie est choisi parmi les mammifères (félidés, canidés, équidés, lagomorphes, rongeurs). De façon avantageuse, le produit selon l'invention est destiné aux chiens et aux chats, cependant, le terme animal de compagnie inclut également les nouveaux animaux de compagnie (NAC) tels que par exemple le furet, les rongeurs (par exemple le lapin, le hamster), le cochon nain.

Dans un aspect de l'invention, le produit selon l'invention est destiné aux chiens. Le produit et les méthodes selon l'invention sont plus particulièrement destinés aux chiens de petite taille ou aux chiens de race ayant une prédisposition aux problèmes cardiaques. De telles races de chien sont par exemple, et de manière non limitative: Caniche, Chihuahua, Bichon, Yorkshire, Cavalier King Charles, Pekingese, Pinscher, Spitz Loup, Spaniels, English Springer Spaniel, Pomeranian, Basset, Beagle, Westie, Whippet, Terriers, Fox terrier, Yorkshire Terrier. Par « petit chien », on entend, dans le cadre de la présente invention, les chiens de moins de 15 ou 10 kilos.

Dans un autre aspect de l'invention, le chien est un grand chien. Par « grand chien », on entend, dans le cadre de la présente invention, les chiens de plus de 30 kilos. Par exemple, et de façon non limitative, un chien de race choisie parmi: Terre-Neuve, Bobtail, Labrador, Berger Allemand, Collie, Doberman, Boxer.

Des chiens qui ne sont pas de race pure mais possèdent des caractéristiques proches desdites races peuvent également être traités en utilisant le produit selon la présente invention.

Dans un mode de réalisation de l'invention, le produit selon l'invention peut être administré en combinaison avec des médicaments usuellement prescrits aux animaux souffrant de pathologies cardiaques, tel que par exemple les antagonistes de l'aldostérone, les inodilatateurs, les inhibiteurs de l'enzyme de conversion, de l'angiotensine comprenant entre autres: Bénazépril, Captopril, Cilazapril, Enalapril, Fosinopril, Imidapril, Lisinopril, Moexipril, Perindopril, Quinapril, Ramipril, Spirapril, Trandolapril.

Le produit selon l'invention est, selon un autre mode de réalisation, un aliment complet, un aliment complémentaire ou diététique à visée santé, c'est-à-dire un produit combinant la notion d'aliment et de médicament dans le cadre d'une alimentation fonctionnelle. Il permet donc par définition de réduire les risques ou de prévenir l'apparition de certaines maladies, à l'aide de substances contenues dans un aliment de consommation courante. Le terme aliment complémentaire ou diététique est utilisé ici pour désigner un aliment qui a un impact positif sur la santé et/ou sur les performances physiques d'un animal, au-delà de la fonction simple de fournir des nutriments. Selon l'invention, les bénéfices observés par le produit concernent notamment les maladies cardiovasculaires.

L'aliment selon l'invention peut ainsi comprendre des substances autres que les ingrédients additionnels détaillés ci-dessus, notamment des extraits de plantes, des matières premières alimentaires (végétales, animales, minérales), des acides aminés, des protéines, des huiles essentielles, des matières grasses (acides gras en particulier), des minéraux, des vitamines ou des substances actives médicamenteuses.

Comme cela est détaillé aux exemples ci-dessous, selon l'invention, le produit est avantageusement utilisé pour le traitement, la prévention ou l'aide au contrôle des maladies cardiovasculaires. Dans le cadre de la présente invention, on entend par « aide au contrôle des maladies cardiovasculaires », un moyen d'apporter un support de la fonction cardiaque chez des animaux, notamment ceux prédisposés à développer des pathologies cardiovasculaires. De préférence, le produit selon l'invention permet le soutien de la fonction cardiovasculaire chez les chiens et en particulier ceux atteints ou prédisposés à développer des maladies valvulaires, en particulier les maladies valvulaires mitrales, les petits chiens ledit produit étant destiné à être donné au chien, âgé en particulier, tous les jours et sur du long terme.

Dans une variante de l'invention, le produit peut être administré de manière quotidienne et ce, tout au long de la vie de l'animal. Dans une autre variante, le produit peut également être administré de manière régulière 1, 2, 3 fois par jour ou tous les 2, 3, 4 jours, 1, 2, 3 fois par semaine ou 1, 2, 3, 4 fois par mois.

Dans une autre variante, le produit peut également être administré sous forme de cures ou cycles de traitement. C'est-à-dire que l'administration du produit, quotidienne ou non, est faite sur une période de temps définie, comprise entre 1 semaine et un an, plus particulièrement pendant 1, 2, 3, 4, 5 ou 6 mois. Dans une variante de l'invention, le produit selon l'invention est administré au moins 2, 3, 4, 5, 6 ou plusieurs fois à l'animal. Dans un mode de réalisation, le produit est administré sous forme de cure de 1, 2, 3, 4, 5, 6 ou plusieurs mois, 1, 2, 3 fois par année. Dans une variante particulière de l'invention, le produit est administré quotidiennement, pour une période comprise entre 1 et 6 mois, sous forme de cure renouvelable une à deux fois par an.

Dans un autre variante particulière, le produit est administré quotidiennement pour une période d'au moins 1, 2, 3, 4, 5 ou 6 mois.

Le produit selon l'invention est administré dans un dose et à une fréquence qui peuvent être choisies et ajustées par l'Homme du Métier. De façon générale le produit de l'invention est administré à une dose comprise entre 0,0001 mg/kg et 350 mg/kg, entre 0,001 mg/kg et 350 mg/kg, entre 0,01 mg/kg et 350 mg/kg, entre 1 mg/kg et 350 mg/kg, entre 10 mg/kg et 350 mg/kg. La quantité totale d'extrait de Grenade contenue dans le produit selon l'invention varie préférentiellement de 0,001 mg/kg à 350 mg/kg, préférablement de 0,01 mg/kg à 50 mg/kg, préférablement encore de 0,1 mg/kg à 35 mg/kg.

De façon alternative, quand le produit selon l'invention est intégré à un aliment complet, la dose quotidienne peut s'exprimer en milligrammes par unité de calorie. Le produit de l'invention est administré dans une quantité comprise entre 0,01 mg/500 kcal et 350 mg/500 kcal, entre 1 mg/500 kcal et 350 mg/500 kcal, entre 10 mg/500 kcal et 350 mg/500 kcal. La quantité totale d'extrait de Grenade contenue dans le produit selon l'invention peut ainsi varier de 0,001 mg/500 kcal à 350 mg/500 kcal, préférablement de 0,01 mg/500 kcal à 50 mg/500 kcal, préférablement de 0,1 mg/500 kcal à 35 mg/500 kcal.

L'invention propose également une méthode pour le traitement ou la prévention des maladies cardiaques chez l'animal, comprenant l'administration d'une composition selon l'invention, dans une quantité suffisante pour soigner ou prévenir les maladies cardiaques. La quantité suffisante est d'au moins 0,0001 mg/kg, au moins 0,001 mg/kg, au moins 0,01 mg/kg. Le traitement est administré sur une durée d'au moins 1, 2, 3 semaines ou 1, 2, 3, 4, 5 ou 6 mois.

Dans le contexte de l'invention, les doses citées s'entendent pour des doses quotidiennes dans le cas d'un traitement quotidien. Ces doses peuvent également être administrées de manière quotidienne uniquement pour une période de temps donnée, lors d'une cure par exemple.

Dans un aspect de l'invention, le produit est utilisé à des fins non-thérapeutiques, par exemple pour améliorer l'état général de l'animal. En effet, le produit selon l'invention peut avoir un effet énergisant, un effet sur la diminution du vieillissement cellulaire et par voie de conséquence réduire l'incidence de maladies et favoriser le rétablissement de l'animal après une maladie ou un accident, ou encore sur l'augmentation de la vitalité. Ces bienfaits seront visibles par une amélioration de l'aspect esthétique de l'animal (pelage brillant, meilleure posture), de son tonus, par l'amélioration de son comportement (interaction avec son maître, sommeil, appétit, plaisir au jeu), l'amélioration de l'état général évitera également l'installation de maladies opportunistes.

### Exemple 1 : Effets in vitro d'agents vasoprotecteurs dans des cellules endothéliales aortiques canines

En utilisant les cellules CnAOECs issues de Cell Applications, Inc, un modèle de culture cellulaire a pu être établi, permettant de tester des fonctions cellulaires essentielles, comme la prolifération et l'apoptose, en réponse à différents stimuli. Le système mis en place permet d'identifier les stimuli pro- et anti-prolifératifs, ainsi que les stimuli pro- et anti-apoptotiques.

Les antioxydants étant les substances d'essai de la phase 2 de l'étude, il est important que les essais mis en place permettent la détection du potentiel anti-prolifératif ainsi que les effets anti-apoptotiques déjà observés dans les cellules endothéliales exposées aux antioxydants suivants : le glutathion (GSH) et la N-acétylcystéine (NAC). Les activités anti-prolifératives et anti-apoptotiques de référence de ces substances ont montré une reproductibilité considérable tout au long de l'étude.

### A. Phase 1 d'établissement des tests d'évaluation des potentiels effets pro/anti-prolifération et pro/anti-apoptotique:

Les cellules CnAOECs ont été cultivées dans des boîtes de cultures recouvertes d'un milieu de culture spécifique. Les cellules CnAOECs ont été marquées avec de la ³H-thymidine et exposées à des agents de référence connus pour moduler l'apoptose et la prolifération dans différents types de cellules endothéliales vasculaires humaines. Ces substances de référence incluent des antioxydants (Glutathion et N-acétylcystéine), des acides gras libres (acide linoléique (ALenS) et γ-linolénique (Lols)), des facteurs de croissance vasculaire (facteur de croissance endothélial vasculaire, VEGF pour « Vascular Endothelial Growth Factor », et facteur de croissance basique des fibroblastes, bFGF pour « basic Fibroblast Growth Factor ») et des sensibilisateurs de l'insuline (Pioglitazone et Rosiglitazone). Après incubation des cellules avec les substances test et de référence, les tests de prolifération et d'apoptose ont été effectués comme décrit plus en détail ci-dessous.

Afin d'établir des tests à la ³H-thymidine évaluant la prolifération et l'apoptose dans des cellules CnAOECs, différents aspects ont été évalués.

### a. Caractérisation des cellules endothéliales aortiques canines et du procédé pour leur mise en culture : conditions expérimentales à l'ensemencement (nombre de cellules requis, conditions de détachement et d'attachement des cellules, taux de croissance, etc.)

Les cellules sont cultivées dans un milieu de culture contenant le facteur de croissance CECGM (Canine Endothélial Cell Growth Médium) et préalablement revêtu de l'agent AFS (Attachement Factor Solution) pour aider à leur adhérence. Pour la mise au point de l'essai, les cellules ont été ensemencées à une densité de 5 000 à 10 000 cellules/cm² dans des boites de culture prétraitées avec de la fibronectine (0,0025% dans un tampon PBS).

### b. Tests de prolifération :

Les cultures cellulaires CnAOECs confluentes ont été réensemencées dans des plaques de culture 96 puits (10 000 cellules par puits). 6h après leur ensemencement, les cellules ont été exposées à la ³H-thymidine (concentration finale: 1µCi/mL) et aux agents de test respectifs pendant 48h, puis ont subi deux étapes de lavage avec du PBS. Après un traitement à la trypsine et un cycle de gel-dégel, l'ADN incorporant la ³H-thymidine a été piégé sur un filtre en fibre de verre en utilisant une « Betaplate TM 96-bien-Cell Harvester » (Wallac, Turku) .

Les quantités de ³H-thymidine incorporées dans les cellules de contrôle (sans ajout d'agent de test) ont été définies comme 100% et les résultats ont été exprimés en fonction de ces cellules contrôle.

Comme le montre la figure 1 qui illustre les effets des différentes substances de référence sur la prolifération des cellules CnAOECs (moyenne de trois expériences indépendantes, effectuées dans quatre puits), l'antioxydant glutathion (GSH), les sensibilisateurs à insuline Pioglitazone (Pio) et Rosiglitazone (Rosi), ainsi que les acides gras libres (acides linoléique (ALenS) et γ-linolénique (Lols)) réduisent considérablement la prolifération des cellules CnAOECs. En revanche et de façon surprenante, les facteurs de croissance endothélial vasculaire (VEGF) et des fibroblastes (bFGF), connus pour augmenter la prolifération des cellules endothéliales humaines, n'ont pas provoqué cette même réponse chez les cellules CnAOECs.

Comme le montre la figure 2 qui illustre la prolifération des cellules CnAOECs, dans un milieu de culture avec du CECGS réduit, bien que la prolifération soit presque complètement bloquée (<2%) par les deux acides gras libres, les effets antiprolifératifs restent dans une fourchette similaire pour le facteur de croissance endothélial vasculaire (bFGF), le facteur de croissance basique des fibroblastes (VEGF), le glutathion (GSH), la Rosiglitazone (Rosi) et la Pioglitazone (Pio). Ainsi, on déduit de ces expériences que le bFGF et le VEGF, contrairement aux modèles de cultures de cellules endothéliales humaines, n'ont pas d'influence sur la prolifération des cellules CnAOECs. Les mêmes proportions de DMSO (1 ‰) et d'éthanol (1,5 ‰), sont utilisées comme solvants pour les acides gras libres ou pour les futures substances à tester.

### c. Essais des substances de référence sur l'apoptose dans les cellules CnAOECs :

Les plaques 60mm contenant des cellules CnAOECs semi-confluentes ont été marquées à la ³H-thymidine (1µCi/mL) pendant 36h. Ces cellules ont ensuite été ensemencées dans des plaques de culture 24 puits (50 000 cellules/puits). Après leur exposition aux agents à tester (24 heures), les cellules ont été traitées avec un tampon de lyse (20 mmol/L TrisHCl, pH 7,5, et 0,4% de Triton X-100 dans du PBS). L'ADN fragmenté (apoptotique) radiomarqué présent dans le surnageant a été compté à l'aide d'un analyseur de liquide scintillant (Liquid Scintillation Analyser). Les résultats ont ensuite été corrélés à la radioactivité totale incorporée dans les cellules (quantifiée après digestion de la suspension restante avec 180µg/mL de DNase). Les valeurs calculées pour les cellules de contrôle (sans ajout d'agent à tester) ont été fixées à 100% et les résultats ont été exprimés en fonction de ces cellules de contrôle. A chaque fois que les substances de référence ont été dissoutes dans de l'éthanol (concentration finale 1,5 ‰), une quantité identique d'éthanol a été ajoutée à tous les autres puits, y compris les puits de contrôle.

### d. Essais d'Apoptose

Les essais d'apoptose ont été menés avec des cellules CnAOECs radio-marquées, à une confluence de 60% et dans des boîtes de culture préalablement traitées à la fibronectine. A cette confluence, l'apoptose des cellules est inhibée après leur exposition aux antioxydants glutathion et N-acétylcystéine. De façon contraire, l'exposition des cellules à des acides gras libres (acide linoléique et acide γ-linolénique) a provoqué une très forte réaction pro-apoptotique.

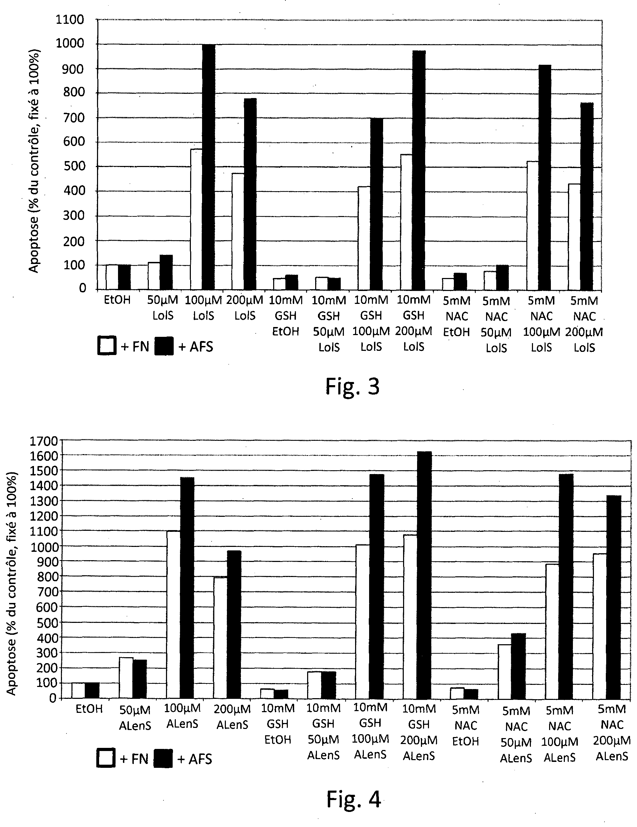

25 µM d'acides gras libres induisant faiblement l'apoptose dans les cellules CnAOECs, les concentrations à utiliser pour les acides gras libres ont donc été fixées à 50, 100 et 200 µM pour les expériences ultérieures. En outre, comme le montrent les figures 3 et 4 qui illustrent l'apoptose des cellules CnAOECs, les effets pro-apoptotiques des acides gras libres ne sont pas complètement bloqués par les antioxydants glutathion (GSH) et N-acétylcystéine (NAC), ni pour l'acide linoléique(ALenS), ni pour l'acide γ-linolénique (LolS).

### Conclusion concernant la phase 1 :

Dans la première phase de l'étude, des protocoles pour la culture des cellules CnAOECs (dans des boîtes de cultures revêtues de fibronectine et dans du milieu de culture pour cellules endothéliales canines) ainsi que pour l'exécution des tests de prolifération et d'apoptose ont été établis.

Les antioxydants glutathion et N-acétylcystéine ainsi que les sensibilisateurs à l'insuline que sont la Pioglitazone et la Rosiglitazone réduisent la prolifération cellulaire de 30 à 70%, tandis que les acides gras libres (acide linoléique et γ-linolénique) bloquent complètement la prolifération endothéliale. Contrairement à leur effet sur les cellules endothéliales vasculaires humaines, bFGF et VEGF n'ont pas d'effet pro-prolifératif sur les cellules CnAOECs.

Les antioxydants glutathion et N-acétylcystéine inhibent significativement l'apoptose dans les cellules CnAOECs, tandis que les acides gras libres provoquent une forte réaction pro-apoptotique. Ces données montrent que le design de l'expérience établi est bien adapté pour tester des substances à la fois pour des effets pro- et anti-apoptotiques.

### B. Phase 2 d'évaluation des substances testées.

### a. Evaluation des substances testées sur la prolifération des cellules CnAOECs :

Avant chaque expérience, une nouvelle solution stock de chacune des substances testées (Taurine, Carnitine, extraits de Grenade et Isoflavones de Soja) a été préparée. La solubilisation des Isoflavones de Soja s'est faite dans du DMSO. Les échantillons expérimentaux, ainsi que ceux des contrôles respectifs (DMSO sans substance à tester) contenaient une concentration finale en DMSO de 1‰. Les substances : Taurine, L-carnitine, concentré d'isoflavones de soja et extrait de pomégranate (Grenade) ont été testées seules ou en association à une concentration finale de 50 µg/mL dans le milieu de culture. Les résultats obtenus ont été comparés obtenus pour les puits de contrôle (sans substances à tester, mais avec la même concentration de solvant, par exemple DMSO).

Pour le mélange des 4 substances à tester, la concentration est de 250 µg/mL, soit 62,5 µg/mL de chaque substance à tester.

La concentration finale de DMSO dans le contrôle ainsi que dans les échantillons expérimentaux exposés au mélange a été ajustée à 1 ‰.

Comme illustré à la figure 5, les substances Taurine (Tau), extrait de Grenade 40% (Pom pour « pomegranate »), L-Carnitine-tartrate (Car), Isoflavones de Soja (Soy) (40% en isoflavones) et un mélange de ces substances (Mix) ont été testés en utilisant les concentrations 50µg/ml pour les substances seules et 62,5pg/ml par substance pour le mélange (soit 250µg/ml) en comparaison avec des concentrations de 10 mmol/L pour le contrôle glutathion (GSH) pour leur efficacité dans la modulation de la prolifération des cellules CnAOECs.

L'évaluation statistique est basée sur trois ou quatre expériences distinctes, chacune d'entre elles étant réalisée avec 2 à 4 puits indépendants.

La Carnitine (Car) et la Taurine (Tau) n'ont pas nettement modulé la prolifération des cellules CnAOECs. Pour ces deux substances, on observe seulement une légère mais significative réduction de la prolifération avec une baisse de 3,75% pour la Carnitine (Car ; taux de renouvellement de 96,25%) et de 4% pour la Taurine (Tau ; taux de renouvellement de 96%).

Pour ce qui concerne la Grenade et les Isoflavones de Soja, on observe une inhibition importante de la prolifération des cellules avec des taux de renouvellement qui ne sont plus que de l'ordre de 10,5% pour l'extrait de pomégranate (Grenade) et de 75% pour le concentré en isoflavones de soja par rapport au contrôle (fixé à 100%). De façon intéressante, le contrôle positif utilisé, le glutathion a réduit la prolifération à seulement 54,75 % du contrôle.

La figure 5 montre également que l'utilisation d'un mélange des 4 substances (Mix) réduit considérablement la prolifération des cellules à 2,75% du contrôle (fixé à 100%). Cet effet inhibiteur significatif est potentiellement dû à l'action antiproliférative de la Grenade (Pom) et des Isoflavones de Soja (Soy), même si dans le mélange, seulement 25% de chacune des quatre substances étaient présents. On observe donc un effet synergique d'au moins deux composés du mélange des 4 substances (Mix) entrainant une action antiproliférative considérable sur les cellules CnAOECs.

La figure 6 présente, dans les mêmes conditions expérimentales, les résultats d'effet dose (1, 50 et 250 µg/ml) pour le pomégranate (Grenade) seul. Cette figure 6 montre que l'effet sur la prolifération cellulaire est obtenu de manière dose dépendant avec le pomégranate (Grenade) seul.

### b. Evaluation des substances testées sur l'apoptose des cellules CnAOECs :

Comme illustré à la figure 7 l'extrait de Grenade à 40% (Pomegranate) et un mélange des substances Taurine, extrait de Grenade, L-Carnitine-tartrate, Isoflavones de Soja (mélange des 4) ont été testés en utilisant les concentrations 1 et 50 µg/mL en comparaison avec 10 mmol/L de l'antioxydant glutathion (GSH). La figure 8 présente une dose réponse de l'effet de l'extrait de Grenade seul aux doses 1, 50 et 250 µg/ml.

L'évaluation statistique est basée sur trois expériences distinctes, chacune d'entre elles étant réalisée avec 2 à 4 puits indépendants.

La modulation de l'apoptose des cellules CnAOECs (variation mesurée en % par rapport au contrôle fixé à 100%) est faite en comparaison avec la substance de référence qu'est le Glutathion (GSH).

L'extrait de Pomégranate (Grenade, en particulier contenant 40% de punicosides) réduit significativement l'apoptose à toutes les concentrations testées et cela de façon dose dépendante. Ainsi, à une concentration de 250 µg/mL, on observe une réduction de 74% comparée aux cellules contrôle (fixé à 100%).

Le mélange des 4 substances (Mix), comme cela est illustré à la figure 7, entraîne une réduction significative de l'apoptose en comparaison avec les cellules contrôles (solvant sans substances test). Néanmoins, ces réductions ne sont pas suffisamment importantes pour être significatives de façon statistique.

### Conclusion concernant la phase 2 :

Comme indiqué ci-dessus, les extraits de Grenade et d'Isoflavones de Soja réduisent la prolifération et l'apoptose de façon dose-dépendante, l'extrait de Grenade présentant les effets les plus importants.

Il est intéressant de noter qu'aux concentrations testées, l'extrait de Grenade présente des effets antiprolifératifs et anti-apoptotiques supérieurs à l'antioxydant glutathion. Le mélange apparait quant à lui réduire considérablement la prolifération à une concentration de 250 µg/mL, tandis que pour l'apoptose, les valeurs moyennes sont inférieures à celles de leurs témoins respectifs.

### Exemple 2 : Evaluation de l'effet protecteur de substances naturelles contre le stress oxydatif à l'aide d'un nouveau test développé sur des cellules endothéliales canines et d'essais de capture des radicaux libres (« radical scavenging assays »)

L'objectif de cet exemple est d'évaluer la protection contre le stress oxydatif induit par l'H₂O₂ dans des cellules endothéliales canines et le potentiel de réduction des radicaux libres par des substances naturelles antioxydantes. Avant ces études, aucun composé n'avait été décrit pour un effet contre le stress oxydatif *in vitro* dans des cellules endothéliales canines. En outre, selon Ram & Hiebert (cf. ci-dessus), l'effet protecteur observé sur les cellules endothéliales aortiques. avec les molécules antioxydantes naturelles pourrait être espèce-dépendant.

Dans les expériences réalisées ci-dessous, les différents produits chimiques et réactifs de culture cellulaire ont été obtenus comme suit :
- les cellules aortiques endothéliales canines (CnAOEC) et le milieu de culture des cellules endothéliales canines (CECGM pour « Canine Endothelial Cell Growth Médium ») proviennent de la société Tebu-Bio™ ;

- les produits Trolox™, ABTS (2,2'-azino-bis(acide 3-éthylbenzthiazoline-6-sulfonique), DPPH (1,1-diphényl-2-picrylhydrazyle), H₂O₂, MTT (3-(4,5-diméthylthiazol-2-yl)-2,5-diphényltetrazolium bromide) proviennent de la société Sigma-Aldrich™ ;
- Le concentré en Isoflavones de Soja est concentré 40% en Isoflavones et provient de la société ADM™ ;
- l'extrait de Grenade concentré à 40% en punicosides provient de Polynat; et enfin
- la L-Carnitine-L-tartrate et la Taurine proviennent de la société Arnaud SAS™.

Des données sur la biodisponibilité sont disponibles chez le chien pour la Taurine, la Carnitine et la Génistéine (l'une des principales Isoflavones de Soja aglycones). Ces données montrent que les concentrations plasmatiques après supplémentation orale peuvent atteindre 50 µg/mL pour la Taurine et la Carnitine et 5 µg/mL pour la Génistéine.

Pour ce qui concerne l'extrait de Grenade, des données ne sont disponibles que pour les humains. Il a été montré dans ces études que l'acide ellagique, l'un des principaux polyphénols de la Grenade alimentaire, peut être trouvé dans le plasma à une concentration d'environ 10 µg/mL après une supplémentation alimentaire en extrait la Grenade.

Les cellules CnAOEC ont été décongelées et cultivées dans un milieu de croissance pour cellules endothéliales canines en accord avec les instructions du fournisseur

Les cellules ont été ensemencées à différentes densités de 2.10⁴ à 5.10⁴ cellules/cm² dans des plaques de 96 puits pour suivi de leur comportement en culture. A 5.10⁴ cellules/cm², les cellules présentent leur aspect typique et ont atteint 100% de confluence après 7 jours de culture. Le comportement et l'apparence des cellules CnAOEC restant inchangés entre les passages 1 à 14, ces cellules ont été utilisées jusqu'au passage 14 au maximum.

Dans cet exemple, les données expérimentales des systèmes acellulaires sont exprimées en moyenne ± SD (pour « standard deviation », c'est-à-dire l'écart-type) et analysées à l'aide du test de Student avec des logiciels commerciaux (SYSTAT, SPSS Inc, Chicago, Ill).

Les données obtenues avec les substances naturelles dans le test à base de cellules CnAOECs ont été analysées statistiquement par un ANOVA simple (one-way ANOVA). Les différences ont été considérées comme significatives à p<0,05.

### a. Mesure de la viabilité cellulaire à l'aide d'un test MTT :

Le test MTT a été utilisé pour évaluer la viabilité des cellules CnAOECs exposées à des traitements cytotoxiques (60 µM de Digitonine ou ajout entre 0,1 et 20% de DMSO ou à des concentrations croissantes (allant de 0,5 à 16 mM) de H₂O₂ pendant 24 heures afin d'induire un stress oxydatif.

Dans ce test, les dommages cellulaires sont évalués 24h après traitement avec la substance cytotoxique par une incubation de 4 heures avec du MTT à 0,5 mg/mL, 37°C et 5% de CO₂. Les plaques sont ensuite centrifugées, et le formazan résultant de la réaction au MTT est solubilisé dans le DMSO. L'absorbance est mesurée à 570 nm, avec une correction à 690 nm.

Le test MTT a été choisi pour mesurer la viabilité cellulaire sur la base' des données obtenues après le traitement de la cellule avec de la Digitonine à 60µM (73% de réduction ± 10%) et avec du DMSO à 10 et 20%.

Les dommages sur les cellules CnAOECs causés par les radicaux libres proviennent d'un traitement à l'H₂O₂ à une gamme de concentration allant de 0,5 à 16 mM.

Les résultats de l'analyse MTT indiquent que la viabilité cellulaire est significativement diminuée après 24h d'exposition.

Ces conditions ont été choisies dans le but d'effectuer le screening des substances naturelles sélectionnées.

### b. Evaluation du potentiel des substances naturelles pour la protection des cellules CnAOECs contre les dommages oxydatifs :

La capacité des substances naturelles à protéger les cellules contre les effets néfastes du stress oxydatif a été évaluée dans les cellules CnAOECs. Les différents échantillons ont été dilués dans du milieu de culture et aucun problème de solubilisation n'a été observé avec chacune des substances testées. Les cellules ont ensuite été prétraitées pendant 8h avec des concentrations croissantes (1, 50, 250µg/mL) d'extrait d'Isoflavones de Soja (Soy), d'extrait de Grenade (Pom), de L-Carnitine (Car), de Taurine (Tau) et du mélange SPCT (Mix), c'est-à-dire le mélange des 4 substances dans lequel chacune des substances est présente respectivement à 0,25, 12,5, et 62,5 µg/mL afin d'obtenir les mêmes concentrations dans l'échantillon final donc 1, 50, 250 µg/mL. Après la période de prétraitement, les cellules ont été soumises à un stress oxydatif (H₂O₂ à 2 mM) pendant 24 heures et la viabilité cellulaire a été évaluée par le test MTT comme décrit précédemment.

Pour ce test, les données sont des moyennes issues de huit expériences différentes ± Ecart type (« Standard deviation », SD). Elles ont été analysées statistiquement par un test ANOVA simple (One way ANOVA Student t-test). Les différences ont été considérées comme significatives lorsque p<0,05.

L'effet protecteur du prétraitement avec des substances naturelles sur la viabilité cellulaire des cellules traitées à l'H₂O₂ a été évalué en utilisant le test MTT. Les données ont montré, comme cela est illustré à la figure 9, à la dose de 50 µg/mL, la Taurine, la L-Carnitine, l'extrait d'Isoflavones de Soja, ne sont pas capables de maintenir la viabilité des cellules CnAOECs pendant l'application d'un stress oxydatif à 2 mM d' H₂O₂.

Cependant, comme cela est illustré à la figure 9 à la dose de 50µg/ml et à la figure 10, pour les concentrations de 1 à 250µg/ml, l'extrait de Grenade montre un effet protecteur fort et significatif sur la viabilité des cellules CnAOECs contre les dommages cellulaires induits par l'H₂O₂ et cela de manière dose-dépendante. En effet, à 50 et 250 µg/mL, l'extrait de Grenade améliore la viabilité des cellules à la suite d'un stress oxydatif de 35% et 82% respectivement. En outre, cet effet protecteur est encore observé lorsque l'extrait de Grenade est mélangé avec les autres substances. En effet, comme illustré à la figure 9, la combinaison des 4 substances à 250µg/mL (correspondant respectivement à une concentration de 62,5 µg/mL pour chaque ingrédient) améliore la viabilité des cellules CnAOEC de 51%.

Le niveau de protection cellulaire induit par le mélange des quatre substances est comparable à la protection induite par l'extrait de Grenade seul.

### c. Dosage Trolox Equivalent Antioxydant Capacity (TEAC) :

Cette méthode est basée sur la capacité des molécules antioxydantes à désactiver le radical ABTS°⁺ à longue durée de vie, en comparaison avec le Trolox, un analogue de la vitamine E soluble dans l'eau, utilisé comme contrôle positif. Le radical ABTS°⁺ est un chromophore bleu-vert avec une absorption caractéristique à 734 nm.

Les échantillons des substances testées et de Trolox sont dilués dans du tampon PBS afin d'obtenir les concentrations choisies. Ensuite, 200 µL d'ABTS°⁺ à une concentration de 7 mM sont ajoutés à 50µL d'échantillon ou de solution Trolox dans des plaques de 96 puits.

L'absorbance des différents échantillons est lue toutes les 2 minutes pendant 6 minutes (temps d'incubation) à température ambiante. Les résultats sont présentés en pourcentage d'inhibition du radical ABTS, et en équivalent Trolox (µM).

Les résultats du test TEAC sont repris dans le tableau A ci-dessous :

**Tableau A:**

| | | Test TEAC | |
|---|---|---|---|
| | | Inhibition ABTS°+ | |
| Echantillon | | % | Ecart-type |
| Contrôle | | 0,00 | 4,09 |
| Isoflavones de Soja (µg/mL) | 1 | 9,04 | 1,94 |
| | 50 | 83,44 | 5,42 |
| | 250 | 91,62 | 2,99 |
| Grenade (µg/mL) | 1 | 27,84 | 4,12 |
| | 50 | 97,37 | 2,75 |
| | 250 | 97,38 | 2,61 |
| L-Carnitine (µg/mL) | 1 | 0,45 | 4,95 |
| | 50 | 0,37 | 4,59 |
| | 250 | 0,92 | 4,19 |
| Taurine (µg/mL) | 1 | -0,50 | 4,02 |
| | 50 | -0,69 | 4,42 |
| | 250 | -0,62 | 4,66 |
| Mélange SPLT. (µg/mL) | 1 | 8,91 | 3,57 |
| | 50 | 97,16 | 2,66 |
| | 250 | 96,79 | 2,82 |

Comme cela est montré au tableau A ci-dessus, les extraits d'Isoflavones de Soja et de Grenade ont montré une forte activité antioxydante. En effet, l'extrait de Grenade a presque complètement quenché, c'est-à-dire affaibli, le radical ABTS à 50 et 250µg/ml. Le radical ABTS est également affaibli, d'une manière dose-dépendante, par 50 et 250µg/mL d'Isoflavones de Soja (83 et 91% de désactivation respectivement). D'autre part, la Taurine et la L-Carnitine n'ont montré aucun effet sur l'inhibition du radical ABTS. Le radical ABTS est également presque complètement inhibé par 50 et 250µg/mL du mélange SPLT (contenant respectivement 12,5 et 62,5µg/mL de chaque ingrédient). Ce pourcentage d'inhibition est équivalent à celui obtenu avec 50 µg/mL d'extrait de Grenade seul.

### d. Test antioxydant DPPH :

Ce test d'activité antiradicalaire est basé sur la capacité des molécules antioxydantes à réduire le radical pourpre stable DPPH (pour 1,1 Diphényl 2 Pycril Hydrazil).

Le test a été réalisé dans des plaques de 96 puits où 190 µL de solution de DPPH (150 µM) dans de l'éthanol ont été mélangés avec 10 µL de solution d'échantillon préparée dans un tampon PBS aux concentrations choisies.

L'absorbance du mélange est lue à 540 nm toutes les 5 minutes pendant 30 minutes à température ambiante. Après réduction, la couleur de la solution disparaît (passage d'une couleur violette à une couleur jaune pâle).

Le pourcentage de réduction (dénommé ici « inhibition » ou « quenching »), reflète la capacité de balayage des radicaux libres de l'échantillon testé.

Le Trolox est également utilisé comme contrôle positif.

Les résultats sont présentés en pourcentage d'inhibition du radical DPPH. Il est à noter qu'avec l'extrait de Grenade à une concentration de 250 µg/mL, un précipité a été observé après l'incubation avec le DPPH, empêchant une lecture pertinente de l'absorbance.

Les résultats du test DPPH sont repris dans le tableau B ci-dessous :

**Tableau B :**

| | | TEST DPPH | |
|---|---|---|---|
| Echantillon | | % DPPH | Ecart-type |
| Contrôle | | 0,00 | 3,74 |
| Isoflavones de Soja (µg/mL) | 1 | 1,65 | 4,93 |
| | 50 | 2,35 | 4,62 |
| | 250 | 10,98 | 4,56 |
| Grenade (µg/mL) | 1 | 11,53 | 7,02 |
| | 50 | 55,26 | 3,39 |
| | 250 | NC | NC |
| L-Carnitine (µg/mL) | 1 | 6,74 | 5,45 |
| | 50 | 4,97 | 5,8 |
| | 250 | 14,94 | 5,09 |
| Taurine (µg/mL) | 1 | 4,95 | 6,03 |
| | 50 | 10,84 | 5,12 |
| | 250 | 25,09 | 4,8 |
| Mélange SPCT (µg/mL) | 1 | 5,26 | 5,29 |
| | 50 | 44,49 | 1,88 |
| | 250 | 50,98 | 5,01 |
| Trolox (µM) | 5 | 4,21 | 3,62 |
| | 10 | 9,91 | 2,75 |
| | 15 | 17,00 | 2,83 |
| | 20 | 23,49 | 3,79 |
| | 25 | 28,6 | 2,75 |
| | 30 | 33,06 | 2,37 |
| | 40 | 41,18 | 1,48 |

Comme cela est montré au tableau B ci-dessus, aussi bien l'extrait de Grenade que le mélange SPCT à 50 µg/mL montrent un effet de balayage significatif sur les radicaux libres DPPH (environ 50%), similaire à l'activité obtenue avec le contrôle Trolox à 40 µM.

L'extrait d'Isoflavones de Soja, la L-Carnitine et la Taurine présentent eux, un effet inhibiteur sur le quenching du radical DPPH de 11%, 15% et 24% respectivement à 250µg/mL.

### Conclusion :

Il a été montré que l'H₂O₂ est en mesure de générer des dommages oxydatifs conduisant à la mort des cellules CnAOECs pour des concentrations supérieures à 2 mM. A des concentrations inférieures à 2 mM, une légère augmentation de l'activité métabolique a été observée. Il a déjà été signalé que, dans certains cas, les cellules survivantes au stress oxydatif induit par l'H₂O₂ développaient une hypertrophie liée à l'activation de différentes voies métaboliques.

Suite à la mise au point du test sur les cellules, le potentiel antioxydant des quatre substances (à savoir la L-Carnitine, la Taurine, les extraits de Grenade et d'Isoflavones de Soja) a été étudié dans des cellules endothéliales canines.

L'extrait de Grenade seul ou en combinaison a pu montrer un effet cytoprotecteur. Cet effet de la Grenade observé sur les cellules CnAOECs « oxydées » est conforme aux résultats précédents établis avec des cellules endothéliales humaines et les macrophages soumis à H₂O₂, montrant que l'extrait de Grenade exerce un effet cytoprotecteur en plus de l'activité antioxydante. L'extrait d'Isoflavones de Soja, la L-Carnitine et la Taurine utilisés seuls n'ont pas montré d'effet protecteur sur les cellules CnAOECs sous l'influence d'un stress oxydatif dans ce modèle. L'absence d'effet cytoprotecteur de la L-Carnitine et de la Taurine suggère que l'activité de protection de ces composés au niveau de la sphère cardiovasculaire pourrait être associée à des mécanismes d'action différents.

La combinaison de substances naturelles (chacune à dose équivalente) a montré un effet protecteur significatif sur les cellules CnAOECs victimes d'un stress oxydatif, augmentant la viabilité cellulaire. Cet effet semble être principalement dû aux propriétés antioxydantes de la Grenade et/ou d'un potentiel effet synergique des substances. Il est important de noter que la présence d'autres substances dans le mélange ne semble pas antagoniser l'activité de la Grenade.

En considérant que la Taurine et la Carnitine sont couramment utilisés chez les chiens atteints de maladies cardiaques, il est important d'établir qu'il n'y a pas d'effet inhibiteur sur d'autres actions antioxydantes quand ils sont combinés. En effet, comme cela est montré dans cette étude, il apparait que la combinaison des différentes substances entraîne une amélioration de l'effet, comme par exemple dans le mélange de 50 µg/mL SPCT (Mix), c'est-à-dire le mélange des 4 substances. Dans ce mélange de 50 µg/mL de SPCT, la Grenade est seulement à 12,5 µg/mL et ce mélange entraîne une augmentation de 41% de la viabilité comparable à la valeur (soit 35%) de la Grenade seule à 50 µg/mL.

Dans le test de balayage des radicaux libres, l'extrait de Grenade a été la seule substance montrant une activité de balayage élevée à la fois sur les radicaux ABTS et DPPH. Ces résultats sont conformes aux résultats d'études antérieures sur l'extrait de Grenade. Les préparations (extraits ou jus) à partir des différentes parties (arilles, écorces ou fruits entiers) d'extrait de Grenade ont révélé des pouvoirs antioxydants divers, évalués avec plusieurs méthodes de balayage des radicaux.

Il semble que l'activité antioxydante de l'extrait de Grenade soit liée à une teneur élevée en punicosides (≥ 40%). Les Isoflavones de Soja n'ont montré une activité de balayage des radicaux directe qu'avec le test TEAC et un faible effet sur les tests DPPH.

Enfin, la L-Carnitine et la Taurine ne montrent aucune activité de balayage avec l'ABTS mais une activité faible (Carnitine) à modérée (Taurine) avec le test DPPH.

Pour résumer, les résultats fournis par des systèmes acellulaires et cellulaires montrent que la Taurine, la L-Carnitine, les extraits de Grenade et les Isoflavones de Soja exercent des activités antioxydantes et cytoprotectrices par des mécanismes d'action différents.

Un traitement multi-composants apparait la meilleure approche pour réduire l'apparition et la progression des maladies cardiaque canines liées au stress oxydatif telles que l'insuffisance cardiaque, après insuffisance mitrale chronique (CMVI), et différentes formes de cardiomyopathies.

### Exemple 3 : Exemple d'une composition orale selon l'invention

| | **Teneur en % (masse/masse totale)** |
|---|---|
| **Ingrédients actifs** | |
| L-Carnitine-L-Tartrate | 21,8 |
| Taurine | 14,7 |
| Extrait de Grenade | 0,9 |
| Extraits de Soja concentrés en Isoflavones | 1,5 |

| **Excipients** | |
|---|---|
| Liant | 32,2 |
| Agent de granulation | 2,8 |
| Lubrifiant | 6,0 |
| Appétent | 20,1 |

### Conclusion :

L'extrait de Grenade et le mélange des quatre substances ont montré une forte action antiradicalaire, antioxydante, anti-proliférative et anti-apoptotique dans les cellules CnAOECs.

Les actions antiradicalaires au niveau des extraits de Grenade et du mélange des quatre substances étant comparables, comme montré dans la figure 11, il semble donc que la Carnitine, la Taurine et les Isoflavones de soja n'aient pas d'effet antagoniste sur l'action de l'extrait de Grenade.

Comme le montrent les résultats des présents exemples, le fait que les activités antioxydantes et antiprolifératives du mélange des quatre substances sur les cellules CnAOECs soient nettement plus forts que ceux de l'extrait de Grenade seul ou de chacune des autres substances testées prises séparément laisse penser qu'il existe un effet synergique associé à ce mélange.

## Revendications

1. Produit pour administration orale destiné à un animal de compagnie, comprenant des extraits de Grenade *Punica granatum*, **caractérisé en ce qu'**il comprend en outre :
- des extraits d'Isoflavones de Soja ;
- de la L-Carnitine ; et
- de la Taurine.

2. Produit selon la revendication 1, **caractérisé en ce qu'**il comprend :
- entre 5 et 15 % en poids du poids total du produit de L-Carnitine ;
- entre 10 et 20 % en poids du poids total du produit de Taurine ;
- entre 0,1 et 5% en poids du poids total du produit d'extraits de Grenade, *Punica granatum* ; et
- entre 0,5 et 5% en poids du poids total du produit d'extraits de Soja concentrés en Isoflavones.

3. Produit selon l'une des revendications 1 à 2 à titre d'aliment complet.

4. Produit selon l'une des revendications 1 à 2 à titre d'aliment complémentaire ou diététique à visée santé.

5. Produit selon l'une des revendications précédentes, pour utilisation dans le traitement ou la prévention des maladies cardiovasculaires.

6. Produit selon l'une des revendications 1 à 4, pour utilisation comme aide au contrôle des maladies cardiovasculaires.

7. Produit selon l'une des revendications 1 à 4, pour utilisation dans le support de la fonction cardiaque dans le cas d'une insuffisance cardiaque chronique.

8. Produit pour administration orale destiné à un animal de compagnie, comprenant des extraits de Grenade Punica granatum, **caractérisé en ce qu'**il comprend :
- des extraits d'Isoflavones de Soja ;
- de la L-Carnitine ; et
- de la Taurine, pour utilisation dans le traitement ou la prévention des maladies cardiovasculaires, et également **caractérisé en ce que** l'animal de compagnie est un canidé ou un félidé.

9. Produit selon l'une des revendications 1 à 7, **caractérisé en ce que** l'animal de compagnie est un canidé ou un félidé.

10. Produit selon l'une des revendications 8 ou 9, **caractérisé en ce que** l'animal est un canidé sélectionné parmi les chiens de race: Caniche, Chihuahua, Bichon, Yorkshire, Cavalier King Charles, Pekingese, Pinscher, Spitz Loup, Spaniels, English Springer Spaniel, Pomeranian, Basset, Beagle, Westie, Whippet, Terriers, Fox terrier, Yorkshire Terrier.

11. Produit selon l'une des revendications 5 à 10, **caractérisé en ce que** le produit est administré quotidiennement pour une période d'au moins 1, 2, 3, 4, 5 ou 6 mois.

12. Produit selon l'une des revendications 5 à 11 **caractérisé en ce que** le produit est administré à une dose comprise entre 0,0001 mg/kg et 350 mg/kg.

13. Produit selon l'une des revendications 1 à 12, sous la forme d'un comprimé, d'une gélule, d'une dragée, d'une poudre, d'un granulé, d'un sirop, d'une suspension, d'une solution, d'une émulsion.

## Patentansprüche

1. Produkt zur oralen Verabreichung, das für ein Haustier vorgesehen ist, umfassend *Punica-granatum-*Granatapfelextrakte, **dadurch gekennzeichnet, dass** es außerdem umfasst:
- Extrakte von Sojaisoflavonen;
- L-Carnitin; und
- Taurin.

2. Produkt nach Anspruch 1, **dadurch gekennzeichnet, dass** es umfasst:
- zwischen 5 und 15 Gew.-% des Gesamtgewichts des Produkts an L-Carnitin;
- zwischen 10 und 20 Gew.-% des Gesamtgewichts des Produkts an Taurin;
- zwischen 0,1 und 5 Gew.-% des Gesamtgewichts des Produkts an Granatapfelextrakten, *Punica granatum*; und
- zwischen 0,5 und 5 Gew.-% des Gesamtgewichts des Produkts an Extrakten von konzentrierten Sojaisoflavonen.

3. Produkt nach einem der Ansprüche 1 bis 2 als Alleinfuttermittel.

4. Produkt nach einem der Ansprüche 1 bis 2 als Ergänzungs- oder diätetisches Futtermittel zu Gesundheitszwecken.

5. Produkt nach einem der vorhergehenden Ansprüche zur Verwendung bei der Behandlung oder Verhinderung von Herz-Kreislauf-Erkrankungen.

6. Produkt nach einem der Ansprüche 1 bis 4 zur Verwendung als Hilfsmittel zur Bekämpfung von Herz-Kreislauf-Erkrankungen.

7. Produkt nach einem der Ansprüche 1 bis 4 zur Verwendung bei der Unterstützung der Herzfunktion im Falle einer chronischen Herzinsuffizienz.

8. Produkt zur oralen Verabreichung, das für ein Haustier vorgesehen ist, umfassend *Punica-granatum-*Granatapfelextrakte, **dadurch gekennzeichnet, dass** es umfasst:
- Extrakte von Sojaisoflavonen;
- L-Carnitin; und
- Taurin,
zur Verwendung bei der Behandlung oder Verhinderung von Herz-Kreislauf-Erkrankungen, und zudem **dadurch gekennzeichnet, dass** das Haustier ein Hund oder eine Katze ist.

9. Produkt nach einem der Ansprüche 1 bis 7, **dadurch gekennzeichnet, dass** das Haustier ein Hund oder eine Katze ist.

10. Produkt nach einem der Ansprüche 8 oder 9, **dadurch gekennzeichnet, dass** das Tier ein Hund ist, der aus den Rassehunden ausgewählt ist: Pudel, Chihuahua, Malteser, Yorkshire, Cavalier King Charles Spaniel, Pekinese, Pinscher, Wolfsspitz, Spaniel, English Springer Spaniel, Pomeranian, Dachshund, Beagle, West Highland White Terrier, Whippet, Terrier, Foxterrier, Yorkshire Terrier.

11. Produkt nach einem der Ansprüche 5 bis 10, **dadurch gekennzeichnet, dass** das Produkt täglich für einen Zeitraum von mindestens 1, 2, 3, 4, 5 oder 6 Monaten verabreicht wird.

12. Produkt nach einem der Ansprüche 5 bis 11, **dadurch gekennzeichnet, dass** das Produkt in einer Dosis verabreicht wird, die zwischen 0,0001 mg/kg und 350 mg/kg liegt.

13. Produkt nach einem der Ansprüche 1 bis 12 in der Form einer Tablette, einer Kapsel, eines Dragées, eines Pulvers, eines Granulats, eines Sirups, einer Suspension, einer Lösung, einer Emulsion.

## Claims

1. Product for oral administration intended for a pet, comprising extracts of *Punica granatum* (pomegranate), **characterised in that** it further comprises:
- Soy Isoflavone extracts;
- L-Carnitine; and
- Taurine.

2. Product according to claim 1, **characterised in that** it comprises:
- between 5 and 15% by weight of the total product weight of L-Carnitine;
- between 10 and 20% by weight of the total product weight of Taurine;
- between 0.1 and 5% by weight of the total product weight of extracts of *Punica granatum* (pomegranate); and
- between 0.5 and 5% by weight of the total product weight of extracts of soy concentrated with Isoflavones.

3. Product according to one of claims 1 to 2 for complete food.

4. Product according to one of claims 1 to 2 for supplementary or nutritional food for health purposes.

5. Product according to one of the preceding claims, for use in the treatment or prevention of cardiovascular diseases.

6. Product according to one of claims 1 to 4, for use as an aid in controlling cardiovascular diseases.

7. Product according to one of claims 1 to 4, for use in cardiac function support in the case of chronic heart failure.

8. Product for oral administration intended for a pet, comprising extracts of *Punica granatum* (pomegranate), **characterised in that** it comprises:
- Soy Isoflavone extracts;
- L-Carnitine; and
- Taurine,
for use in the treatment or prevention of cardiovascular diseases, and also **characterised in that** the pet is a Canidae or a Felidae.

9. Product according to one of claims 1 to 7, **characterised in that** the pet is a Canidae or a Felidae.

10. Product according to one of claims 8 or 9, **characterised in that** the animal is a Canidae selected from the pedigree dogs: Poodle, Chihuahua, Bichon, Yorkshire, Cavalier King Charles, Pekingese, Pinscher, Keeshond, Spaniels, English Springer Spaniel, Pomeranian, Basset, Beagle, Westie, Whippet, Terriers, Fox Terrier, Yorkshire Terrier.

11. Product according to one of claims 5 to 10, **characterised in that** the product is administered daily for a period of at least 1, 2, 3, 4, 5 or 6 months.

12. Product according to one of claims 5 to 11 **characterised in that** the product is administered at a dose between 0.0001 mg/kg and 350 mg/kg.

13. Product according to one of claims 1 to 12, in the form of a tablet, hard capsule, sugar-coated tablet, powder, granule, syrup, suspension, solution, emulsion.
